# EUROPÄISCHE PATENTSCHRIFT

(11) **EP 1 334 115 B1**
(45) Veröffentlichungstag und Bekanntmachung des Hinweises auf die Patenterteilung: **16.05.2007**
(21) Anmeldenummer: 01992714.4
(22) Anmeldetag: 31.10.2001
(51) Int. Cl.: C07J 73/00, A61K 31/58, A61P 35/00

(54) **PHENANTHRIDINDERIVATE UND PHENANTHRIDIN ENTHALTENDE ANTITUMORALE ARZNEIMITTEL**
PHENANTHRIDINE DERIVATIVES AND ANTITUMORAL MEDICAMENTS CONTAINING PHENANTHRIDINE
DERIVES DE PHENANTHRIDINE ET MEDICAMENTS ANTITUMORAUX CONTENANT DE LA PHENANTHRIDINE

(30) Priorität: 02.11.2000 DE 10054337
(43) Veröffentlichungstag der Anmeldung: 13.08.2003
(73) Patentinhaber: Clement, Bernd, D-24106 Kiel (DE)
(72) Erfinder: Clement, Bernd, D-24106 Kiel (DE)
(86) Internationale Anmeldenummer: PCT/EP2001/012665
(87) Internationale Veröffentlichungsnummer: WO 2002/036607

(56) Entgegenhaltungen:
- WO-A-97/14683
- JANIN Y L ET AL: "SYNTHESIS AND EVALUATION OF NEW 6-AMINO-SUBSTITUTED BENZOUCPHENANTHRIDINE DERIVATIVES" JOURNAL OF MEDICINAL CHEMISTRY, AMERICAN CHEMICAL SOCIETY. WASHINGTON, US, Bd. 36, Nr. 23, 1993, Seiten 3686-3692, XP000652079 ISSN: 0022-2623

## Beschreibung

Die Erfindung betrifft neue Phenanthridinderivate sowie Arzneimittel zur antitumoralen Therapie und Prophylaxe, die diese Phenanthridinderivate enthalten. Als Stand der Technik soll auf die WO 97-14683 verwiesen werden in der bereits Phenanthridinderivate und deren Syntheseverfahren beschrieben wurden.

Während die Synthese von Phenanthridinderivaten aus dem Stand der Technik bereits seit langem bekannt ist und eine Vielzahl verschiedener Synthesezyklen existiert, ist die pharmakologische Wirkung dieser Verbindungen erst vor kurzem entdeckt worden. So beschreibt die WO 97/14683 erstmalig die antitumorale Aktivität dieser Verbindungen. Die antitumorale Aktivität der dort beschriebenen Verbindungen für die medizinische Applikation ist dagegen begrenzt.

Ausgehend hiervon ist es die Aufgabe der vorliegenden Erfindung neue Phenanthridinderivate anlehnend an die in der WO 97/14683 beschriebenen Verbindungen anzugeben, die sich durch eine, gegenüber dem Stand der Technik, erhöhte antitumorale Aktivität auszeichnen.

Die Aufgabe wird in bezug auf die Phenanthridinderivate durch die Merkmale des Anspruchs 1, in bezug auf die Arzneimittel durch die Merkmale des Anspruchs 7 gelöst. Die Unteransprüche zeigen vorteilhafte Weiterbildungen auf. Die Verwendung der Phenanthridine wird gemäß den Ansprüchen 12 bis 16 beschrieben.

Erfindungsgemäß konnten neue Phenanthridinderivate mit ausgezeichneten antitumoralen und zytotoxischen Eigenschaften geliefert werden. Diese weisen die allgemeine Formel I 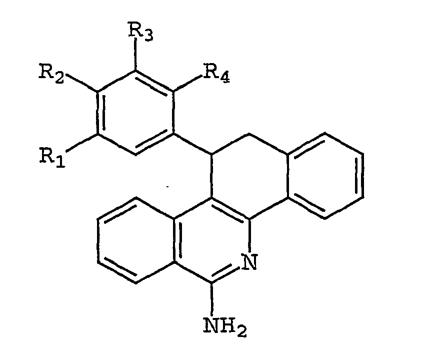 und II 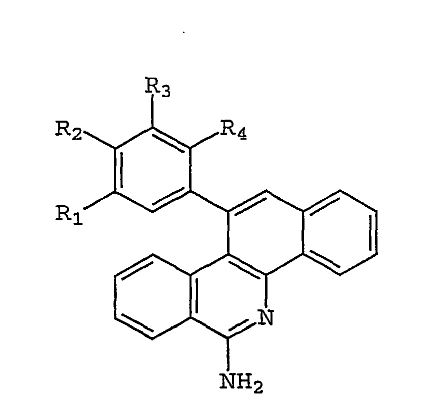 auf. Die Reste R₁ bis R₄ sind dabei so gewählt, daß drei dieser Reste Methoxygruppen sind und ein Rest ein Wasserstoff ist.

Überraschenderweise konnte gezeigt werden, daß derart spezifisch substituierte Phenanthridinderivate eine bisher aus dem Stand der Technik weder bekannte noch zu erwartende antitumorale Aktivität aufweisen. Dies bezieht sich sowohl auf die subkutane wie auf die intraperitoneale Anwendung. Dahingegen weisen die aus der WO 97/14683 bekannten Verbindungen im Bereich der subkutanen wie intraperitonealen Anwendung eine wesentlich geringere Aktivität auf.

Die vorstehend beschriebenen Phenanthridinderivate weisen ausgezeichnete antitumorale, antimikrobielle, antifungizide, antivirale und antiinflammatorische Eigenschaften auf. Zur Untersuchung der pharmakologischen Eigenschaften wurden die Verbindungen in einem "in-vivo-Antitumor-Screening" des National Cancer Institute (NCI), Bethesdal, Maryland, USA, untersucht. Die Verbindungen wurden dabei gegen zwölf humanpathogene Tumorzelllinien von sechs verschiedenen Krebsarten (nicht-kleinzelliger Lungenkrebs, Melanom, Brustkrebs, Eierstockkrebs, Dickdarmkrebs, ZNS-Krebs) getestet.

In bevorzugter Weise liegen die Derivate in ionischer Form, besonders bevorzugt als Salz vor. Die erfindungsgemäßen Phenanthridinderivate führen dabei ohne weiteres physiologisch annehmbare Salze. Solche Salze sind z.B. Salze mit anorganischen und organischen Säuren, z.B. die Hydrochloride, Hydrobromide und Sulfate. Besonders gut geeignete Salze von organischen Säuren werden mit aliphatischen Mono- und Dicarbonsäuren gebildet. Beispiele hierfür sind Acetate, Maleate und Fumarate.

Folgende Verbindungen sind dabei aufgrund der höchsten antitumoralen Aktivität bevorzugt:
A) 6-Amino-11-(3',4',5'-trimethoxyphenyl)benzo[c]-phenanthridiniumperchlorat mit einem Grundgerüst der Formel III, 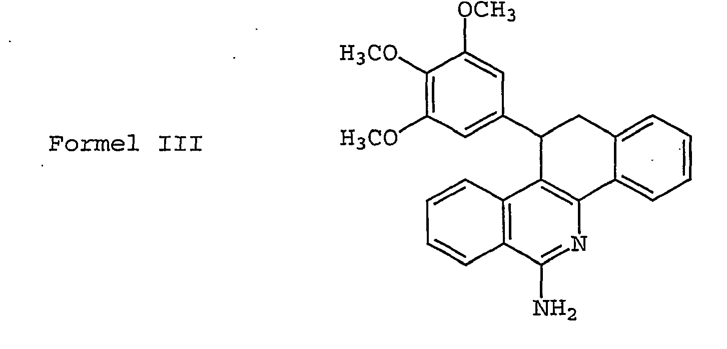
B) 6-Amino-11,12-dihydro-11-(2',3',4'-trimethoxyphenyl)benzo[c]phenanthridiniumchlorid mit einem Grundgerüst der Formel IV, 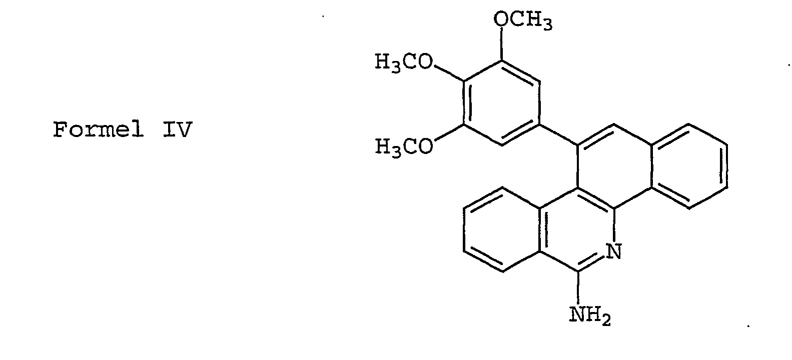
C) 6-Amino-11,12-dihydro-11-(3',4',5'-trimethoxyphenyl)benzo[c]phenanthridiniumchlorid mit einem Grundgerüst der Formel V,

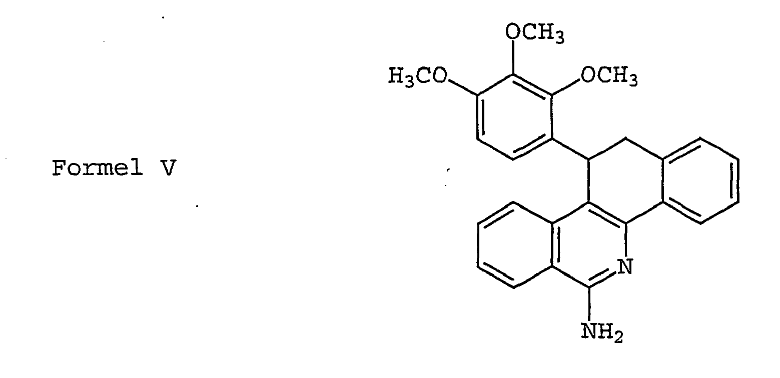

Die Erfinduung betrifft ebenso Arzneimittel, die die vorstehend beschriebenen Phenanthridinderivate enthalten. Diese Arzneimittel enthalten dabei mindestens eines der Phenanthridinderivate mit mindestens einem pharmazeutischen Trägerstoff. Vorzugsweise enthält das Arzneimittel zusätzlich noch weitere Verdünnungsmittel. Bevorzugt werden als Phenanthridinderivate dabei die Verbindungen der allgemeinen Formel III, IV und/oder V eingesetzt. Die erfindungsgemäßen Verbindungen können oral, topisch oder parenteral sowie in Form von Suppositoren verabreicht werden. Der bevorzugte Verarbreichungsweg ist die perorale Verabreichung. Sie kann in Form der Base oder als physiologisch annehmbares Salz verabreicht werden. Sie wird im allgemeinen mit einem pharmazeutisch annehmbaren Träger oder Verdünnungsmittel vermengt, um ein Arzneimittel zu ergeben. Für die orale Verabreichung kann das Arzneimittel am zweckmäßigsten in Form von Kapseln oder Tabletten vorliegen, die auch Tabletten mit verzögerter Freisetzung sein können. Die Arzneimittel können auch in Form von Dragees oder in Sirupform vorliegen. Geeignete topische Zubereitungen sind z.B. Salze, Lotionen, Cremes, Pulver und Sprays. In gleicher Weise betrifft die Erfindung die Verwendung mindestens einer der oben genannten Phenanthridinderivate zur Herstellung eines Arzneimittels zur antitumoralen Therapie und Prophylaxe.

Die weiteren Beispiele und Figuren sollen beispielhaft weitere Details und Eigenschaften der erfindungsgemäßen Verbindungen erläutern.

### Beispiel 1

### Synthese von 6-Amino-11-(3',4',5'-trimethoxyphenyl)benzo[c]phenanthridiniumperchlorat

Zu einer Lösung von 4.85 mmol (2.0 g) 6-Amino-11,12-dihydro-11-(3',4',5'-trimethoxyphenyl)benzo[c]phenanthridin in 120 ml Dioxan wird eine Lösung von 19.4 mmol (4.63 g) DDQ in 100 ml Dioxan gegeben und 16 h unter Rückfluß erhitzt. Das erkaltete Reaktionsgemisch wird in 500 ml ges. Natriumhydrogencarbonatlösung hydrolysiert und 30 min kräftig gerührt. Die wäßrige Phase wird dreimal mit je 200 ml Ether extrahiert, die vereinigten organischen Phasen einmal mit 300 ml ges. Natriumhydrogencarbonatlösung und dreimal mit je 250 ml Wasser gewaschen. Anschließend wird die organische Phase ggf. über Natriumsufalt getrocknet und auf ca. 150 ml einrotiert. Zur Fällung des 6-Aminobenzo[c]phenanthridiniumperchlorates wird die organische Phase über Nacht mit 10 ml 70proz. Perchlorsäure oder ggf. mit 20 ml einer Lösung von 70proz. Perchlorsäure, Ethanol und Ether (1/1/1) kräftig gerührt. Der braune Niederschlag wird abgesaugt, mit wenig Ether gewaschen, aus Methanol umkristallisiert und 24 h im Ölpumpenvakuum getrocknet.

Ausb.: 1.12 g (44% d.Th.), dunkelbraune Nadeln, Schmp.: 296 °C
- C₂₆H₂₃N₂O₇Cl (510.93): Ber.: C 61.12 H 4.54 N 5.48
O 21.92 Cl 6.94
Gef.:
C 61.10 H 4.55
N 5.40

**¹H-UMR (DMSO-d₆, 300 MHz):**
**δ/ppm (TMS)** = 3.73 (s, 6H, 2 -OCH₃), 3.79 (s, 3HOCH₃), 6.77 (s, 2H, ArH), 7.65-7.95 (m,6H, ArH), 8.14 (d, 1H, ArH), 8.67 (d, 2H, ArH), 9.82 (br, s, 2H, -NH₂), 12.95 (br, s, 1H, =N⁺-H).

**¹³C-NMR (DMSO-d₆ 75 MHz):**
**δ/ppm (TMS)** = 56.1 (2C, 2 -OCH₃), 60.3 (-OCH₃), 106.3 (2C), 121.1, 126.1, 127.6, 127.9, 128.1, 128.4, 128.7, 128.9, 133.2 (11C, C-1, -2, -3, -4, -7, -8, - 9, -10, -12, -2', -6'), 114.9, 118.1, 121.8, 130.4, 132.3, 134.2, 136.6, 137.4, 137.7, 153.5 (2C), 154.6, (12C, C-4a, -4b, -6, -6a, -10a, -10b, -11, -12a, -1', -3', -4',-5').

**MS (EI):**
**m/z (%)** = 410 (M⁺ der Base, 100), 395 (M⁺ -CH₃, 20), 380 (M⁺ -2 CH3, 3), 363 (6), 352 (9), 335 (8), 320 (9), 305 (8), 292 (15), 281 (10).

**IR (KBr):**
**v/cm⁻¹** = 3355, 3234, 1683, 1595, 1517.

### Beispiel 2

### Synthese von 6-Amino-11,12-dihydro-11-(2',3',4'-trimethoxyphenyl)benzo[c]phenanthridiniumchlorid

Zu einer Lösung von 88 mmol (9.86 g) Kalium-*tert-*butylat in 90 ml DMPU wird eine Lösung von 80 mmol (9.36 g) 2-Methylbenzonitril und 40 mmol (7.85 g) 2,3,4-Trimethoxybenzaldehyd in 60 ml DMPU langsam unter Stickstoffatmosphäre bei 30 °C zugetropft. Anschließend wird das Reaktionsgemisch 3-4 h bei 35-40 °C gerührt und dann in einer Lösung von 80 mmol (8.8 g) Ammoniumchlorid in 400 ml Eiswasser vorsichtig hydrolysiert. Die wäßrige Phase wird dreimal mit je 200 ml Dichlormethan extrahiert und die organische Phase auf etwa 100 ml einrotiert. Zur Fällung des 6-Amino-11,12-dihydro-11-(2',3',4'trimethoxyphenyl)benzo[c]phenanthridiniumchlorids wird die organische Phase über Nacht mit 20 ml 5N Salzsäure kräftig gerührt. Der farblose Niederschlag wird abgesaugt, mit wenig Dichlormethan gewaschen, aus Methanol/Wasser (3:1) umkristallisiert und die farblosen Kristalle im Ölpumpenvakuum 24 h getrocknet.

Ausb.: 11.40 g (64% d.Th.), farblose Kristalle, Schmp.: 283 °C
- C₂₆H₂₅N₂O₃Cl (448.95): Ber.: C 69.56 H 5.61
N 6.24 O 10.69 Cl 7.90
Gef.: C 69.51 H 5.48 N 6.21

**¹H- NMR (DMSO-d₆, 300 MHz):**
**δ/ppm (TMS)** = 3.08 (d, 1H, H-12a, ²J _{(H-12a,H-12b)} = 15.8 Hz), 3.52 (mc, 1H, H-12b), 3.59 (s, 3H, -OCH₃), 3.79 (s, 3H, -OCH₃), 4.13 (s, 3H, -OCH₃), 5.04 (d, 1H, H-11, ³J _{(H-11,H-12b)} =7.0 Hz), 6.04 (d, 1H, ArH), 6.31 (d, 1H, ArH), 7.35 (mc, 1H, ArH), 7.44 (mc, 1H, ArH), 7.73 ) (d, 2H, ArH), 7.93 (mc, 1H, ArH), 8.62 (d, 1H, ArH), 9.80 (br, s, 2H, -NH₂), 14.04 (br, s, 1H, ≡N⁺ -H).

**¹³C-NMR (DMSO-d₆ 75 MHz):**
**δ/ppm (TMS)** = 30.2 (C- 11), 34.8 (C- 12), 55.5 (-OCH₃), 60.3 (-OCH₃), 61.2 (-OCH₃), 107. 1, 123.4, 123.8, 125.8, 126.2, 127.1, 128.1, 129.2, 130.1, 135.0 ( 10C, C-1, -2, -3, -4, -7, -8, -9, -10, -5',--6'), 117.4, 117.5, 121-8, 127.4, 133.0, 135.2, 135.7, 141.7, 150.3, 152.3, 155.3 (11C -4a, -4b, -6, -6a, -10a, -10b, -12a, -1', -2', -4').

**IR (KBr):**
**v/cm⁻¹ =** 3258, 3072, 2934, 2834, 1642, 1620, 1600, 1570, 1554.

### Freisetzen der Base:

Das 6-Amino-11,12-dihydro-11-(2',3',4'-trimethoxyphenyl)benzo[c]phenanthridiniumchlorid wird mit 20 ml konz. Ammoniak und 200 ml Diethylether 1 h kräftig gerührt. Die organische Phase wird abgetrennt und unter kräftigem Rühren in 350 ml Petrolether (50-70) gegeben. Der Niederschlag wird abgesaugt, anschließend wird der Diethylether abrotiert und weiterer Niederschlag abgesaugt. Der gesamte Rückstand wird dreimal mit wenig Petrolether gewaschen und die farblosen Kristalle des 6-Amino-11,12-dihydro-11-(2',3',4'-trimethoxyphenyl)benzo[c]phenanthridins 24 h im Ölpumpenvakuum getrocknet.

Schmp.: 137 °C
- C₂₆H₂₄N₂O₃ (412.49): Ber.: C 75.71 H 5.86 N 6.79
O 11.64
Gef: C 75.65 H 5.88 N 6.65

**¹H- NMR (DMSO-d₆, 300 MHz):**
**δ/ppm (TMS)** = 3.04 (d, 1H, H-12a , ²J_{(H-12a,H-12b)} = 15.5 Hz), 3.57 (mc, 1H, H-12b), 3.63 (s, 3H, -OCH₃), 3.89 (s, 3H, -OCH₃), 4.18 (s, 3H, -OCH₃), 5.15 (d, 1H, H-11, ³J_{(H-11,H-12b)} = 7.3 Hz), 5.20 (br, s, 2H, -NH₂), 6.12 (mc, 2H, ArH), 7.04 (d, 1H, ArH), 7.19 (mc, 1H, ArH), 7.31 -7.41 (m, 2H, ArH), 7.54 (mc, 1H, ArH), 7.80 (mc, 2H, ArH), 8.36 (d, 1H, ArH).

**¹³C-NMR (DMSO-d₆ 75 MHz):**
**δ/ppm (TMS)** = 31.3 (C-11), 39.9 (C- 12), 55.6 (-OCH₃), 60.7 (-OCH₃), 61.3 (-OCH₃), 106.8, 122.9, 123.1, 123.7, 124.5, 125.4, 126.8, 128.2, 130.4 (10C, C-1, C-2, C-3, C-4, C-7, C-8, C-9, C-10, C-5', C-6'), 118.5, 119.3, 129.3, 135.7, 135.9, 136.9, 142.5, 144.9, 151.2, 156.0 (11C, C-4a, C-4b, C-6, C-6a, C-10a, C-10b, C-12a, C-1', C-2', C-3', C-4').

**MS (EI):**
**m/z (%) =** 412 (M⁺, 95), 397 (M⁺-CH₃, 10), 381 (M⁺ -OCH₃, 37), 245 (M⁺-2,3,4-Trimethoxyphenyl, 100), 231 (59), 201 (10), 168 (5), 153 (7).

**IR (KBr):**
**v/cm⁻¹** = 3426, 3310, 3178, 2918, 2815, 1630, 1595, 1557.

### Beispiel 3

### Synthese von 6-Amino-11,12-dihydro-11-(3',4',5'-trimethoxyphenyl)benzo[c]phenanthridiniumchlorid

Zu einer Lösung von 88 mmol (9.86 g) Kalium-tert-butylat in 90 ml DMPU wird eine Lösung von 80 mmol (9.36 g) 2-Methylbenzonitril und 40 mmol (7.85 g) 3,4,5-Trimethoxybenzaldehyd in 40 ml DMPU langsam unter Stickstoffatmosphäre bei 30 °C zugetropft. Anschließend wird das Reaktionsgemisch 4 h bei 35-40 °C gerührt und dann in einer Lösung von 80 mmol (8.8 g) Ammoniumchlorid in 400 ml Eiswasser vorsichtig hydrolysiert. Die wäßrige Phase wird zweimal mit je 250 ml Dichlormethan extrahiert und die organische Phase auf etwa 200 ml einrotiert. Zur Fällung des 6-Amino-11,12-dihydro-11-(3',4',5'-trimethoxyphenyl)benzo[c]phenanthridiniumchlorids wird die organische Phase über Nacht mit 10 ml konz. Salzsäure kräftig gerührt. Der helle Niederschlag wird abgesaugt, mit wenig Dichlormethan gewaschen, aus Methanol/Wasser (3:1) umkristallisiert und die farblosen Kristalle im Ölpumpenvakuum 24 h getrocknet.

Ausb.: 10.7 g (60% d.Th.), farblose Kristalle, Schmp.: 272 °C
- C₂₆H₂₅N₂O₃Cl (448.95): Ber.: C 69.56 H 5.61 N 6.24
O 10.69 Cl 7.90
Gef.: C 69.48 H 5.53 N 6.17

**¹H-NMR (DMSO-d₆, 300 MHz):**
**δ/ppm (TMS)** = 3.13 (d, 1H, H-12a, ²J_{(H-12a,H-12b)} = 15.7 Hz), 3.50 (s, 6H, 2 -OCH3),3.5* (mc, 1H, 1H-12b), 3.52 (s, 3H, -OCH₃), 4.83 (d, 1H, H-11, ³J_{(H-11,H-12b)} = 6. 5 Hz), 6.34 (s, 1H, ArH), 7.27 (d, 1H, ArH), 7.38 (mc, 1H, ArH), 7.45 (mc, 1H, ArH); 7.75 (mc, 1H, ArH), 7.98 (mc, 1H, ArH), 8.04 ( d, 1H, ArH), 8.34 (d, 1H, ArH), 8.62 (d, 1H, ArH), 9.55 (br, s, 2H, -NH2), 13.87 (br, s, 1H, ≡N⁺-H).
*) überlagert durch Signale der Methoxygruppen.

**¹³C-NMR (DMSO-d₆ 75 MHz):**
**δ/ppm (TMS)** = 35.9 (C-12), 36.7 ( C-11), 55.6 (2C, 2 -OCH₃), 59.8 (-OCH₃), 104.6 (2C), 123.4, 124.5, 126.1, 127.1, 128.0, 129.2, 130.1, 135.1 (10C, C-1, -2,-3,-4,-7,-8,-9,-10,-2', -6'), 117.5, 117.9, 127.6, 132.7, 135.2, 135.9, 136.2, 137.1, 152.6 (2C), 155.3 (11C, C-4a, -4b, -6, -6a, - 10a, - 10b, - 12a, - 1', -3', -4', -5').

**IR (KBr):**
**v/cm⁻¹** = 3388, 3156, 2940, 2842, 1665, 1633, 1598.

### Freisetzen der Base:

Das 6-Amino-11,12-dihydro-11-(3',4',5'-trimethoxyphenyl)benzo[c]phenanthridiniumchlorid wird mit 10 ml konz. Ammoniak und 100 ml Diethylether 1 h kräftig gerührt. Die organische Phase wird abgetrennt und unter kräftigem Rühren in 150 ml Petrolether (50-70) gegeben. Der Niederschlag wird abgesaugt, anschließend wird der Diethylether abrotiert und weiterer Niederschlag abgesaugt. Der gesamte Rückstand wird zweimal mit wenig Petrolether gewaschen und die farblosen Kristalle des 6-Amino- 11,12-dihydro-11-(3, 4',5'-trimethoxyphenyl)benzo[c]phenanthridins 24 h im Ölpumpenvakuum getrocknet.
farblose Kristalle, Schmp: 182 °C
- C₂₆H₂₄N₂O₃ (412.49): Ber.: C 75.71 H 5.86 N 6.79
O 11.64
Gef.: C 75.70 H 5.81 N 6.68

**¹H-NMR (DMSO-d₆, 300 MHz):**
**δ/ppm (TMS)** = 3.07 (d, 1H, H-12a, ²J_{(-12aH,H-12b)} = 15.7 Hz), 3.54* (mc, 1H, H-12b), 3.55 (s, 6H, 2 -OCH₃), 3.70 (s 3H, -OCH₃), 4.66 (d, 1H, H-11, ³J_{(H-11,H-12b)} = 6.7 Hz), 4.74 (br,s, 2H, -NH₂), 6.25 (s, 2H, ArH), 7.05 (d. 1H, ArH), 7.19 (mc, 1H, ArH),7.30-7.40 (m, 2H, ArH), 7.55 (mc, 1H, ArH), 7.82 (mc, 2H, ArH), 8.33 (d, 1H, ArH).
*) überlagert durch Signale der Methoxygruppen

**¹³C-NMR (DMSO-d₆ 75 MHz):**
**δ/ppm (TMS)** = 37.8 (C-12), 39.9 (C-11), 56.5 (2C, 2 -OCH₃), 61.3 (-OCH₃), 105.3 (2C), 123.8, 124.3, 125.1, 126.1, 127.6, 128.9, 129.1, 131.0 (10C, C -1, -2, -3, -4, -7, -8, -9, -10, -2', -6'), 118.4, 118.9, 135.5, 136.0 (2C), 137.0, 139.7, 144.7, 153.5 (2C), 156.1 (11C, C-4a, -4b, -6, -6a, -10a, -10b, -12a, -1', -3', -4', -5').

**MS (EI):**
**m/z (%)** = 412 (M⁺, 50), 397 (M⁺-CH₃, 8), 245 (M⁺ -3,4,5-Trimethoxyphenyl, 68), 206 (9), 201 (6), 168 (3), 153 (11), 77 (8), 43 (100).

**IR (KBr):**
**v/cm⁻¹** = 3495, 3382, 2943, 2848, 1632, 1592, 1508.

### Pharmakologische Testergebnisse der Verbindungen aus den Beispielen 1 bis 3

Zur Untersuchung der pharmakologischen Eigenschaften wurden diese Verbindungen (BP-7, BP-8, BP-D8) sowie das 6-Amino-11-(2',4'-dimethoxypenyl)benzo[c]phenanthridiniumperchlorat (BP-D3) [PCT/WO 97/14683)]in einem "*in vivo*-hollow fiber assay" des National Cancer Institute (NCI), Bethesda, Maryland, USA untersucht.

Die Verbindungen wurden dabei gegen 12 humanpathogene Tumorzellinien von 6 verschiedenen Krebsarten (nicht-kleinzelliger Lungenkrebs, Melanom, Brustkrebs, Eierstockkrebs, Dickdarmkrebs, ZNS-Krebs) getestet. Diese Zellinien wurden in Polyvinylidenfluorid-Hohlfasern, die für kleine Moleküle durchlässig sind, kultiviert und Nacktmäusen implantlert, wobei jedem Tier jeweils drei Hohlfasern intraperitoneal und subkutan implantiert wurden, d.h. pro Versuchstier konnten drei Tumorzellinien in zwei physiologischen Bereichen untersucht werden. Die Tiere wurden viermal im Abstand von jeweils 24 h mit einer Dosis der Testsubstanz behandelt. Dabei wurde jede Verbindung in zwei Konzentrationen eingesetzt. Nach Ende der Behandlung wurden die Hohlfasern zurückgewonnen und die Zahl der vitalen Tumorzellen bzw. der Proteinbiomasse photometrisch über einen MTT-Formazan-Assay ermittelt, um so die Wachstumshemmung durch die Phenanthridine zu bestimmen. Als Referenz dienten Mäuse mit in Hohlfasern präparierten Tumorzellkulturen, die nur mit dem Lösungsmittel behandelt wurden.

Die Bewertung der Testergebnisse erfolgt über ein vom NCI vorgegebenes Punktesystem. Jede Gabe einer Testsubstanz, die zu einer Abnahme des Tumorzellwachstums von mindestens 50% führt, wird mit zwei Punkten bewertet. Die maximale Punktzahl für eine Verbindung beträgt somit 96 Punkte (12 Zellinien * 2 Implantationsregionen * 2 Konzentrationen der Testsubstanz * 2 Punkte). Zur weiteren Spezifizierung werden die Ergebnisse an subkutan und intraperitoneal implantierten Hohlfasern zudem separat bewertet. Vom NCI werden drei Kriterien vorgegeben, von denen eine Testsubstanz im "in vivo-hollow fiber assay" mindestens ein Kriterium erfüllen muß, um als für weiterführende Testungen geeignet angesehen werden zu können:
(a) Die Summe der Punkte aus intraperitonealen und subkutanen Proben muß größer als 20 sein.
(b) Die Summe der Punkte der subkutanen Proben muß größer als 8 sein.
(c) Bei mindestens einer Zellinie muß die vitale Tumorzellmasse nach der Behandlung geringer als der Startwert sein (zelltötende Eigenschaften).

Bei den untersuchten Phenanthridinen zeigte das 6-Amino-11-(3',4',5'-trimethoxyphenyl)benzo[c]phenanthridiniumperchlorat (BP-D7) im "in vivo-hollow fiber assay" die größte Aktivität und erfüllt die Kriterien (a), (b) und (c) (siehe Tab. 1)

**Tab. 1: Testergebnisse der Phenanthridine im "in vivo-hollow fiber assay" des National Cancer Institute.**

| **Substanz** | **Punkte (gesamt)** | **Punkte (intra-peritoneal)** | **Punkte (subkutan)** | **zelltötende Eigenschaften** |
|---|---|---|---|---|
| BP-D7 | 28 | 14 | 14 | ja |
| BP-8 | 20 | 10 | 10 | ja |
| BP-7 | 16 | 6 | 10 | ja |
| BP-D3 | 10 | 2 | 8 | ja |

| | | | | |
|---|---|---|---|---|
| BP-D7 6-Amino-11-3',4',5'-trimethoxyphenyl)benzo[c]phenanthridiniumperchlorat BP-8 6-Amino-11,12-dihydro-11-(2',3',4'-trimethoxyphenyl)benzo[c]phenanthridiniumchlorid BP-7 6-Amino-11,12-diliydro-11-(3',4',5'-trimethoxyphenyl)benzo[c]phenanthridiniumchlorid BP-D3 6-Amino-11-2',4'-dimethoxyphenyl)benzo[c]phenanthridiniumperchlorat | | | | |

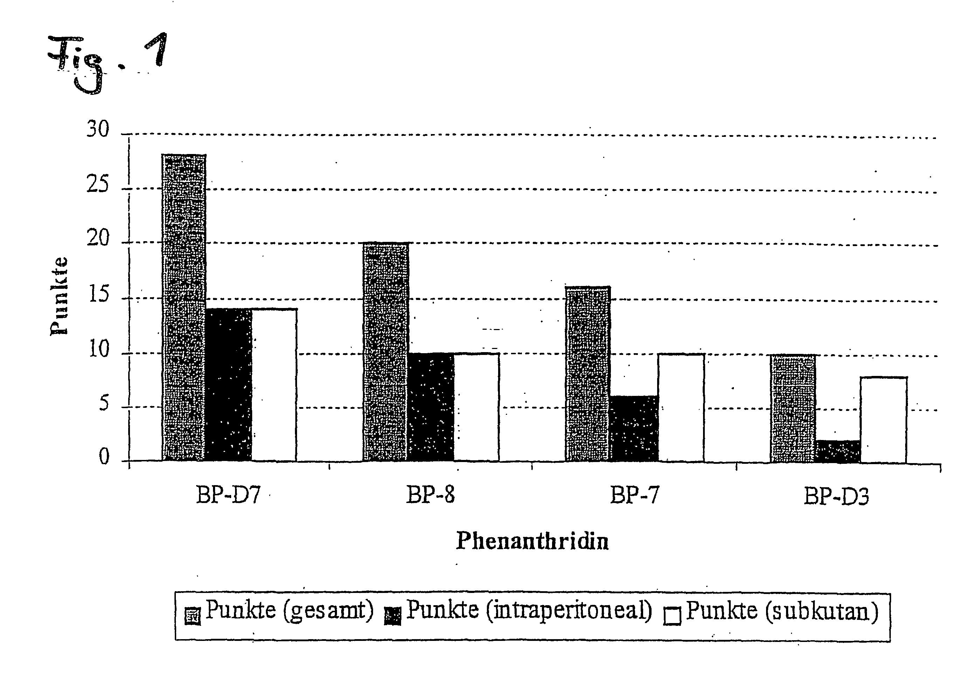

Das 6-Amino-11,12-dihydro-11-(2',3',4'-trimethoxyphenyl)benzo[c]phenanthridiniumchlorid (BP-8) und das 6-Amino-11,12-dihydro- 11-(3',4',5'-trimethoxyphenyl)benzo[c]phenanthridiniumchlorid (BP-7) erfüllen die Kriterien (b) und (c), das 6-Amino-11-(2',4'-dimethoxyphenyl)benzo[c]phenanthridiniumperchlorat (BP-D3) hingegen erfüllt ausschließlich das Kriterium (c). Die Phenanthridine BP-D7, BP-8 und BP-7 erzielen somit deutlich höhere Punktzahlen sowohl in den einzelnen Kategorien (subkutan und intraperitoneal) als auch in der Gesamtpunktzahl als die Verbindung BP-D3. In der Figur 1 sind die Testergebnisse für diese Phenanthridine in Form einer Balkengrafik wiedergegeben.

## Patentansprüche

1. Phenanthridinderivate der allgemeinen Formel I 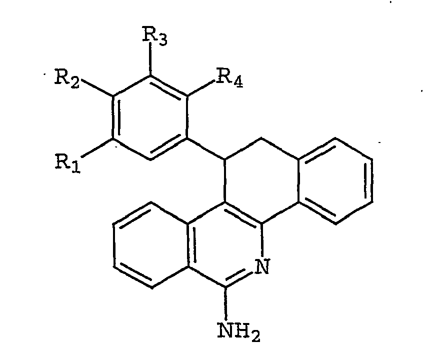 und II 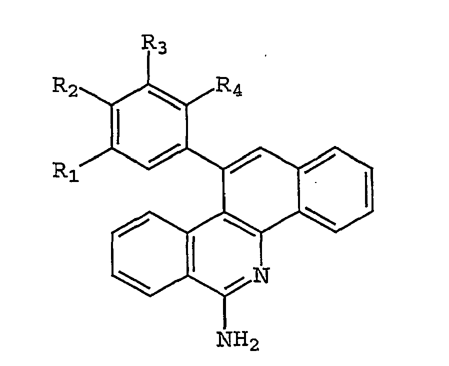 wobei drei Reste R₁ bis R₄ eine Methoxygruppe und der andere Rest Wasserstoff ist.

2. Phenanthridinderivate nach Anspruch 1, **dadurch gekennzeichnet, dass** die Derivate in ionischer Form vorliegen.

3. Phenanthridinderivate nach Anspruch 1, **dadurch gekennzeichnet, dass** die Derivate als Salz vorliegen.

4. Phenanthridinderivate nach mindestens einem der Ansprüche 1 bis 3 der Formel III

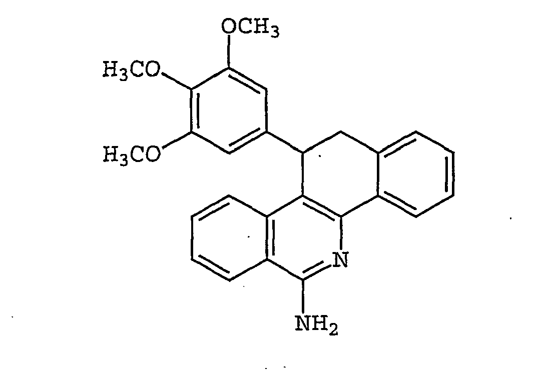

5. Phenanthridinderivate nach mindestens einem der Ansprüche 1 bis 3 der Formel IV

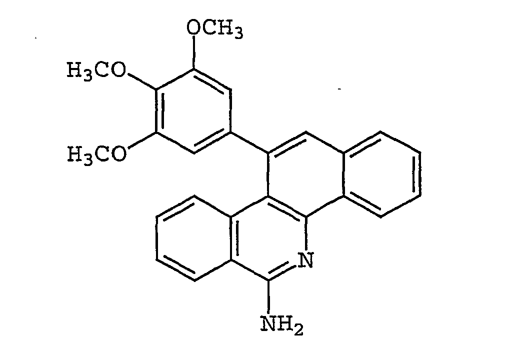

6. Phenanthridinderivate nach mindestens einem der Ansprüche 1 bis 3 der Formel V

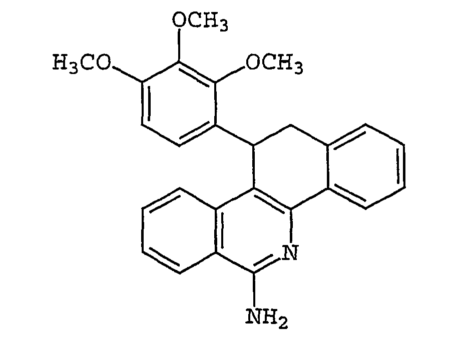

7. Arzneimittel zur antitumoralen Therapie und Prophylaxe, enthaltend mindestens eines der Phenanthridinderivate der vorhergehenden Ansprüche und einen pharmazeutischen Trägerstoff.

8. Arzneimittel nach Anspruch 7 enthaltend mindestens eines der Phenanthridinderivate der allgemeinen Formeln III bis V.

9. Arzneimittel nach mindestens einem der Ansprüche 7 oder 8, enthaltend mindestens ein Verdünnungsmittel.

10. Arzneimittel nach mindestens einem der Ansprüche 7 bis 9,
**dadurch gekennzeichnet, dass** das Arzneimittel als Formulierung für die orale Anwendung vorliegt.

11. Arzneimittel nach mindestens einem der Ansprüche 7 bis 9,
**dadurch gekennzeichnet, dass** das Arzneimittel als Formulierung für die topische Anwendung vorliegt.

12. Verwendung mindestens eines der Phenanthridinderivate der Ansprüche 1 bis 6 zur Herstellung eines Arzneimittels zur antitumoralen Therapie und Prophylaxe.

13. Verwendung nach Anspruch 12,
**dadurch gekennzeichnet, dass** als Phenantridinderivate mindestens eine Verbindung der allgemeinen Formeln III bis V eingesetzt wird.

14. Verwendung nach mindestens einem der Ansprüche 12 oder 13,
**dadurch gekennzeichnet, dass** zusätzlich pharmazeutische Trägerstoffe und/oder Verdünnungsmittel verwendet werden.

15. Verwendung nach mindestens einem der Ansprüche 12 bis 14,
**dadurch gekennzeichnet, dass** das Arzneimittel als Formulierung für die orale Anwendung hergestellt wird.

16. Verwendung nach mindestens einem der Ansprüche 12 bis 14,
**dadurch gekennzeichnet, dass** das Arzneimittel als Formulierung für die topische Anwendung hergestellt wird.

## Claims

1. Phenanthridine derivatives of the general formula I 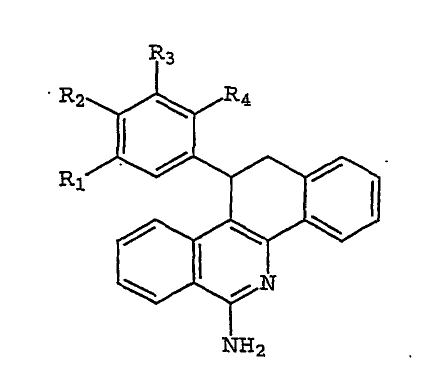 and II 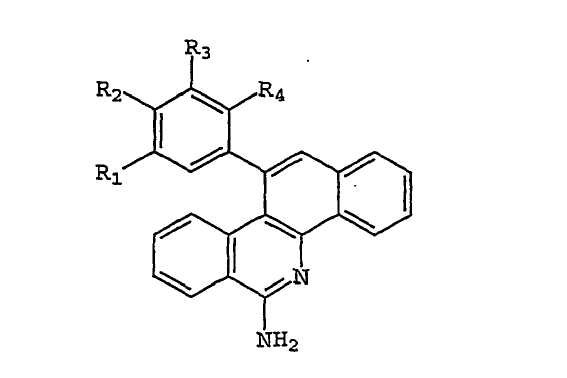 wherein three of R1 to R4 are methoxy and one is hydrogen.

2. Phenanthridine derivatives according to claim 1, **characterised in that** the derivatives exist in an ionic form.

3. Phenanthridine derivatives according to claim 1, **characterised in that** the derivatives exist as salts.

4. Phenanthridine derivatives according to at least one of the claims 1 to 3 having the formula III

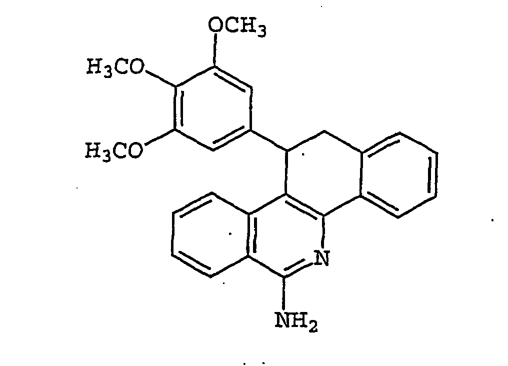

5. Phenanthridine derivatives according to at least one of the claims 1 to 3 having the formula IV

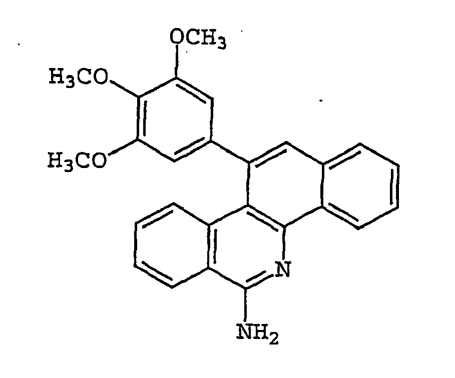

6. Phenanthridine derivatives according to at least one of the claims 1 to 3 having the formula V

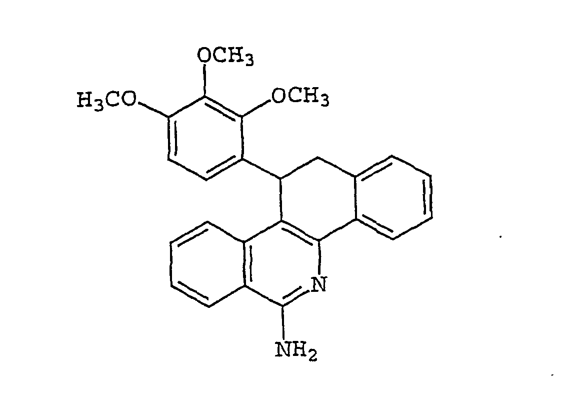

7. A pharmaceutical composition for antitumoral therapy and tumor prophylaxis containing at least one phenanthridine derivative according to the previous claims and at least one pharmaceutically acceptable carrier.

8. A pharmaceutical composition according to claim 7 wherein at least one phenanthridine derivative is of formula III to V

9. A pharmaceutical composition according to claim 7 or 8 containing at least one dilution medium.

10. A pharmaceutical composition according to at least one claim 7 to 9, **characterised in that** the pharmaceutical composition is an oral composition.

11. A pharmaceutical composition according to at least one claim 7 to 9, **characterised in that** the pharmaceutical composition is a topical composition.

12. A method of treating a tumor or of tumor prophylaxis which comprises administering at least one phenanthridine derivative according to claim 1 to 6.

13. The method according to claim 12 **characterised in that** at least one phenanthridine derivative according to formula III to V is administered.

14. The method according to claim 12 or 13 **characterised in that** pharmaceutically acceptable carrier and/or dilution media are used.

15. The method according to claim 12 to 14 **characterised in that** the pharmaceutical composition is an oral composition.

16. The method according to claim 12 to 14 **characterised in that** the pharmaceutical composition is a topical composition.

## Revendications

1. Dérivé de phenanthridine de formule générale I 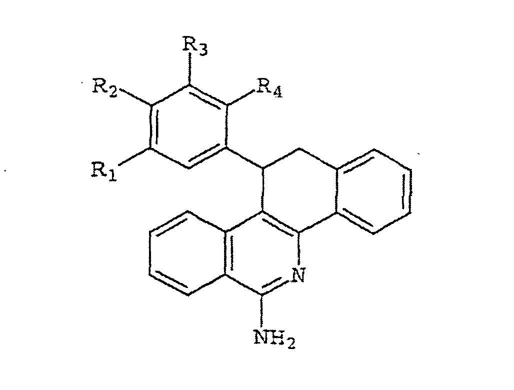 et II 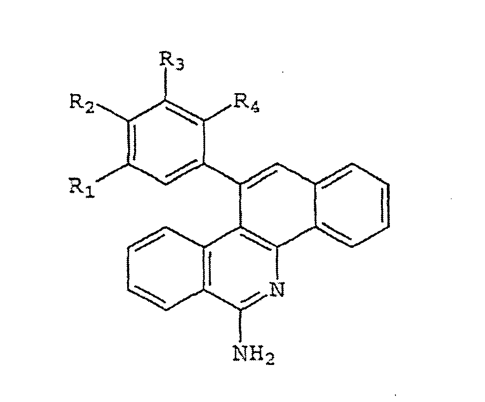 ou trois de R1 a R4 sont methoxique et un est hydrogène.

2. Dérivé de phenanthridine selon revendication 1, charactérisée par un dérivé ionique.

3. Dérivé de phenanthridine selon revendication 1, charactérisée par un dérivé du sel.

4. Dérivé de phenanthridine selon du moins l'une de revendication 1 à 3 représenteé par formule générale III

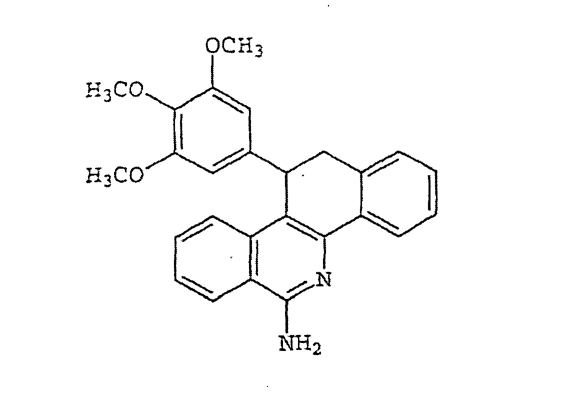

5. Dérivé de phenanthridine selons du moins l'une de revendication 1 à 3 répresenteé par formule générale IV

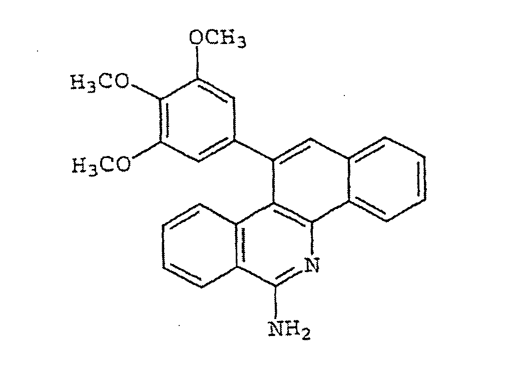

6. Dérivé de phenanthridine selons du moins l'une de revendication 1 á 3 répresenteé par formule générale V

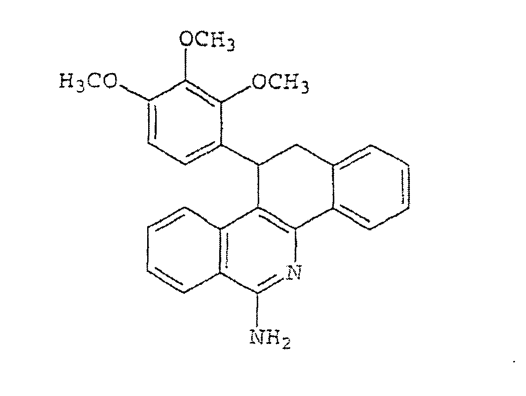

7. Préparation pharmaceutique pour la thérapie antitumorale et tumor prophylaxie contenit du moins un dérivé de phenanthridine de revendications précédentes et du moins un detenteur pharmaceutique acceptable.

8. Préparation pharmaceutique selon revendication 7 contient l'une de dérivé de phenanthridine de la formule générale III à V.

9. Préparation pharmaceutique selon revendication 7 et 8 contient du moins une media de diluer.

10. Préparation pharmaceutique selon revendication 7 à 9 **caractérisée par** une préparation orale.

11. Préparation pharmaceutique selon revendication 7 à 9 **caractérisée par** une préparation topique.

12. Une méthode de traitement une tumeur ou tumeur prophylaxie par l'administration du moins un dérivé de phenanthridine selon revendication 1 à 6.

13. Une méthode selon revendication 12 **caractérisée par** du moins un dérivé de phenanthridine selon la formule générale III à V est administré.

14. Une méthode selon du moins l'une de revendications 12 ou 13 **caractérisée en ce que** un supplémentaire detenteur pharmaceutique acceptable et/ou une media de diluer est utilisé.

15. Une méthode selon du moins l'une de revendications 12 à 14 **caractérisée par** une préparation pharmaceutique orale.

16. Une méthode selon du moins l'une de revendications 12 à 14 **caractérisée par** une préparation pharmaceutique topique.
